# FASCICULE DE BREVET EUROPEEN

(11) **EP 2 455 131 B1**
(45) Date de publication et mention de la délivrance du brevet: **23.01.2013**
(21) Numéro de dépôt: 11178359.3
(22) Date de dépôt: 22.08.2011
(51) Int. Cl.: A61N 1/05, A61N 1/362

(54) **Sonde de stimulation d'une cavité gauche du coeur, implantable dans le réseau coronarien**
Sonde zur Stimulation einer linken Herzkammer, die in das Netz der Herzkranzgefäße implantiert werden kann
Probe for stimulating a left cavity of the heart which can be implanted in the coronary network

(30) Priorité: 19.11.2010 FR 1059521
(43) Date de publication de la demande: 23.05.2012
(73) Titulaire: Sorin CRM SAS, 92140 Clamart (FR)
(72) Inventeur: Ollivier, Jean-François, 91190 Villiers Le Bacle (FR)
(74) Mandataire: Dupuis-Latour, Dominique

(56) Documents cités:
- US-A1- 2003 167 082
- US-A1- 2008 082 136
- US-A1- 2009 088 827
- US-A1- 2009 157 136
- US-A1- 2010 114 284

## Description

L'invention concerne les "dispositifs médicaux implantables actifs" tels que définis par la Directive 90/385/CEE du 20 juin 1990 du Conseil des communautés européennes, notamment les implants permettant de surveiller en continu le rythme cardiaque et délivrer si nécessaire au coeur des impulsions électriques de stimulation ou de resynchronisation.

Elle concerne plus précisément les sondes de stimulation cardiaque destinées à être implantées dans le réseau coronarien du coeur pour permettre la stimulation d'une cavité gauche, ventricule ou oreillette.

A la différence des cavités droites pour lesquelles il suffit d'implanter des sondes endocavitaires via le réseau veineux périphérique droit, la mise en place de sondes permanentes dans une cavité du coeur gauche impliquerait des risques opératoires importants, par exemple le risque de passage de bulles vers le réseau vasculaire cérébral situé en aval du ventricule gauche.

Pour cette raison, lorsque l'on veut stimuler une cavité gauche, on choisit le plus souvent d'introduire une sonde non pas dans la cavité à stimuler mais dans le réseau coronarien, la sonde étant pourvue d'une électrode qu'il conviendra d'appliquer contre la paroi de l'épicarde et d'orienter vers le ventricule gauche ou l'oreillette gauche, selon le cas.

Une sonde de ce type est par exemple le modèle *Situs LV,* commercialisé par Sorin CRM (Clamart, France) et décrit dans le EP 0 993 840 A1 (ELA Medical).

L'introduction d'une telle sonde se fait par le sinus coronaire débouchant dans l'oreillette droite, donc par voie endocavitaire. La sonde est ensuite orientée et poussée le long du réseau des veines coronaires jusqu'au site de stimulation choisi. Cette intervention est très délicate compte tenu des particularités du réseau veineux et de ses voies d'accès, avec notamment le passage de valvules et tortuosités ainsi que la diminution progressive de diamètre du conduit au fur et à mesure de la progression de la sonde dans la veine coronaire sélectionnée.

Une fois atteint la veine cible, il faudra en premier lieu que le chirurgien s'assure de la stabilité mécanique de la sonde dans la veine.

Un autre problème réside dans la difficulté qu'il y a à trouver un site de stimulation satisfaisant, à obtenir un bon contact électrique de l'électrode de stimulation contre le tissu de l'épicarde, et à maintenir ce contact malgré les variations ou sollicitations diverses au cours du temps.

Il faut enfin que le chirurgien s'assure que le point de stimulation choisi ne génère pas de stimulation phrénique.

Pour remédier à ces difficultés, il a été proposé de disposer plusieurs électrodes le long du corps de sonde pour augmenter les chances d'un compromis acceptable, en donnant éventuellement au corps de sonde une conformation particulière. Le chirurgien peut ainsi sélectionner, parmi les diverses électrodes présentes sur le corps de sonde, celle procurant la meilleure efficacité sur le plan électrique et hémodynamique.

Une telle sonde à électrodes multiples est décrite notamment dans le EP 1 938 861 A1 (ELA Medical).

Ces sondes permettent en particulier de mettre en oeuvre le concept de "repositionnement électronique", visant à diriger ou rediriger le champ électrique entre différentes électrodes disposées le long de la sonde de stimulation de la cavité gauche et/ou avec une des électrodes de la sonde de stimulation de la cavité droite. Cette technologie permet de gérer les micro-déplacements ou évolutions du comportement hémodynamique (re*verse modeling)* simplement par reprogrammation du générateur par télémétrie à travers la peau, sans réintervention chirurgicale lourde.

La contrepartie de cette solution est une complexité grandissante de la structure de la sonde, la multiplication du nombre d'électrodes entraînant une multiplication du nombre de composants, et donc de liaisons électriques, ou bien le recours à des circuits de multiplexage pour la sélection des diverses électrodes présentes sur une même sonde.

Le US 2009/157136 A1 décrit une technique de recherche du site de stimulation optimal au moyen d'un cathéter temporaire de *mapping* à introduire dans le sinus coronaire. Ce cathéter est soit un tube flexible ouvert à ses deux extrémités, soit un fil-guide. Dans l'un ou l'autre cas, il est doté en partie distale de multiples électrodes électriquement indépendantes, et en partie proximale d'un connecteur de liaison à un système d'acquisition qui permettra d'identifier le meilleur site de stimulation par un algorithme basé sur le mouvement cardiaque.

Une sonde définitive multiélectrode permanente classique (de diamètre standard est de 4,5 à 6 F) est ensuite placée jusqu'à la position sélectionnée, via le guide (technique dite OTW, *Over The Wire* ou "filoguidage" classique), ou dans le tube, du cathéter temporaire.

Une autre tendance des développements récents en matière de sondes de stimulation du ventricule gauche est la réduction du diamètre de la partie implantable dans le réseau coronaire, jusqu'à un diamètre de 4 French (1,33 mm).

La taille du corps de sonde est en effet un facteur directement lié aux capacités de guidage contrôlé de la sonde dans le réseau coronaire veineux, de manière à y sélectionner des sites de stimulation particuliers situés dans certaines veines collatérales. Ces sites sont atteints au moyen d'un cathéter de sous-sélection de veine servant à mettre en place un mandrin de guidage jusqu'au site choisi. Une fois la veine sélectionnée et le mandrin posé, le chirurgien fait progresser le corps de sonde qu'il glisse sur le mandrin, ce dernier jouant le rôle d'un fil-guide de faible diamètre guidant axialement le corps de sonde jusqu'à l'emplacement choisi (technique dite OTW, *Over The Wire* ou "filoguidage").

Ces diverses solutions présentent toutefois une double limitation, qui est celle :
- de la finesse de la sonde, dont le diamètre ne permet pas d'atteindre les veines collatérales les plus profondes : ainsi, pour la sonde *Situs* précitée, celle-ci présente un diamètre de 2,2 mm (6,6 French) et nécessite un introducteur de diamètre 7 French ; et
- du positionnement correct et du bon maintien en contact électrique de l'électrode contre le tissu à stimuler (paroi de l'épicarde).

Si les techniques précitées de sonde multi-électrodes et de repositionnement électronique permettent de s'affranchir (plus ou moins bien) de la seconde limitation, elles aggravent en revanche la première limitation, dans la mesure où la multiplication des électrodes et des composants ou conducteurs internes implique nécessairement un accroissement du diamètre du corps de sonde et diminue sa flexibilité, rendant difficile voire impossible le passage des tortuosités.

Les solutions proposées sont donc toujours un compromis entre ces deux contraintes.

Le but de l'invention est de s'affranchir de ces deux limitations, en proposant une sonde de stimulation du ventricule gauche dont la partie active :
- présente un très faible diamètre, permettant d'exploiter toute la longueur de la veine et d'utiliser de façon optimale toutes les veines présentes dans la zone basale, en particulier pour éviter le risque de stimulation phrénique qui augmente généralement lorsque le sonde est trop distale ;
- garantisse un contact électrique d'excellente qualité et durable avec les tissus à stimuler ;
- multiplie ou élargit les zones de stimulation, permettant ainsi (contrairement aux sondes traditionnelles) de stimuler simultanément plusieurs zone de l'épicarde en améliorant de ce fait les chances d'une resynchronisation optimale.

Le but de l'invention est également de proposer une telle sonde qui soit de structure simple (donc peu coûteuse à fabriquer, et présentant une fiabilité maximale) et qui s'affranchisse des problèmes liés à la conception et à l'emploi des sondes à électrodes multiples, dont on a exposé plus haut la complexité structurelle et fonctionnelle.

En d'autres termes, l'invention a pour but de proposer une sonde permettant, une fois le site de stimulation choisi et évalué, de garantir une stabilité optimum et pérenne de l'électrode de stimulation sur ce site. L'invention permet également de dissocier la problématique de la stabilité de celle de la performance électro-hémodynamique. En effet, comme on le verra, la stabilité sera assurée par la partie distale de la sonde (vis silicone ou préforme), tandis que la stimulation sera assurée par un microcâble télescopique doté d'une ou de plusieurs zones stimulantes continues ou disjointes.

En particulier, si l'on peut garantir que l'électrode restera quoi qu'il advienne placée au site ou elle a été initialement implantée, on évitera les déplacements ultérieurs de la sonde et il ne sera plus nécessaire de pallier les conséquences d'un tel déplacement par des techniques complexes telles que le repositionnement électronique ou la sélection parmi des électrodes multiples.

Essentiellement, l'invention propose une sonde de stimulation destinée à être implantée dans une veine du réseau coronarien pour la stimulation d'une cavité gauche du coeur, comportant les éléments connus d'après le US 2009/157136 A1 précité, c'est-à-dire comprenant un câble télescopique en matériau conducteur, comportant à son extrémité distale une partie libre active comprenant une pluralité de régions dénudées distinctes, ces régions dénudées étant destinées à venir en contact avec la paroi d'une veine cible du réseau coronaire, de manière à constituer ainsi un réseau d'électrodes de stimulation. Le câble comprend en outre, côté proximal, des moyens de couplage à un générateur de dispositif médical implantable actif tel qu'un stimulateur cardiaque ou resynchroniseur.

De façon caractéristique de l'invention, le câble télescopique est un microcâble, dont le diamètre est compris entre 0,5 et 2 French, et ce microcâble est formé d'une pluralité de brins toronnés ensemble, dont au moins certains sont des brins incorporant une âme en matériau radio-opaque de type platine-iridium ou tantale enveloppée dans une gaine en matériau mécaniquement endurant de type NiTi ou inox, ou inversement. En outre, les régions dénudées distinctes sont des régions dénudées du microcâble, et forment un réseau d'électrodes de stimulation reliées électriquement ensemble.

Le microcâble de faible diamètre (typiquement 1 à 2 French) va servir à canuler des veines de très faible diamètre, non exploitées à ce jour du fait de la taille excessive des sondes coronaires permanentes.

Cette double contrainte conduit à la structure de type microcâble caractéristique de l'invention, structure sans lumière interne avec plusieurs micro-fils tressés ensemble, seule configuration capable d'assurer à la fois l'endurance (à l'encontre des mouvements cardiaques) et la résistance aux sollicitations liées à la pose.

Ce microcâble est introduit dans le réseau coronarien via une sonde porteuse (sans capacité particulière de *mapping)* elle aussi permanente, préalablement placée dans la veine.

Le faible diamètre est la caractéristique essentielle qui permet de répartir la surface de l'électrode monopolaire via de multiples fenêtres aménagées le long du corps du microcâble. Ceci permet de stimuler de façon permanente une large zone de la paroi cardiaque via cette microsonde monopolaire (pour des diamètres de 1 à 2 French, il ne serait pas concevable de prévoir un isolement individuel de chaque brin capable de résister aux contraintes d'abrasion entre brins).

Diverses formes de réalisation peuvent être envisagées.

L'extrémité distale du microcâble peut en particulier être pourvue d'une préforme bi- ou tridimensionnelle, dont les dimensions externes à l'état de repos sont typiquement comprises dans un cube de 1 à 90 mm de côté. Le microcâble peut être ainsi posé seul, et maintenu en place notamment par sa seule préforme distale.

Dans ce cas, le placement dans la veine est réalisé par des moyens classiques de type cathéter/sous-cathéter ou cathéter d'accès cérébral.

Dans une forme particulière de mise en oeuvre, l'ensemble de l'invention est complété par un corps de sonde comportant une gaine creuse en matériau déformable, avec une lumière centrale ouverte à ses deux extrémités et dans laquelle le microcâble est disposé à coulissement en s'étendant sur toute la longueur du corps de sonde et au-delà de l'extrémité distale de celui-ci, la partie du microcâble émergeant au-delà de l'extrémité distale du corps de sonde formant ladite une partie libre active.

Pour assurer le maintien dans la veine, l'extrémité distale du corps de sonde peut être pourvue de moyens de retenue comprenant au moins un relief formé sur le corps de sonde, notamment un relief hélicoïdal avec un filet s'enroulant autour du corps de sonde, présentant localement un diamètre accru par rapport au diamètre propre du corps de sonde, notamment un diamètre accru inférieur ou égal à 7 French.

Le corps de sonde comporte de préférence une partie principale prolongée côté distal par une partie de transition de diamètre inférieur à celui de la partie principale, notamment un diamètre de la partie principale inférieur ou égal à 6 French et un diamètre de la partie de transition inférieur ou égal à 5 French.

Dans une variante de mise en oeuvre, la sonde comprend un corps de sonde commun et une pluralité de microcâbles télescopiques distincts, dont chacun est logé dans le corps de sonde et est apte à coulisser dans celui-ci, les parties libres actives respectives des différents microcâbles émergeant du corps de sonde en des emplacement distincts, espacés longitudinalement sur le corps de sonde.

La surface totale exposée de la (des) région(s) dénudée(s) de la partie libre active du microcâble est de préférence d'au moins 1 mm², avantageusement comprise entre 4 et 6 mm², avec une longueur de partie libre active réglable entre 1 et 200 mm.

Dans un premier mode de réalisation de l'invention, la partie libre active du microcâble comporte une pluralité de régions dénudées distinctes s'étendant en succession le long de cette partie libre active. Avantageusement, ces régions dénudées distinctes sont séparées les unes des autres par des parties de tube en matériau électriquement non conducteur, enveloppant et gainant le microcâble entre deux régions dénudées consécutives, et les régions dénudées portent des bagues tubulaires en matériau électriquement conducteur, serties sur le microcâble. Le matériau électriquement conducteur des bagues tubulaires serties sur le microcâble est de préférence un matériau radio-opaque.

En variante, le microcâble comprend une structure multifilaire revêtue par un matériau isolant, notamment par du parylène, dans lequel les régions dénudées sont formées en ménageant des ouvertures par ablation le long du microcâble, et dépôt éventuel de nitrure de titane sur les régions dénudées ainsi formées.

La longueur en direction longitudinale de chaque région dénudée est typiquement comprise entre 0,5 et 10 mm.

Dans un second mode de réalisation de l'invention, la partie libre active du microcâble comporte au moins une région dénudée hélicoïdale s'étendant le long de cette partie libre active. Le microcâble peut notamment comporter, sur au moins une portion de la partie libre active, un toron formé d'une pluralité de brins torsadés présentant en surface une pluralité correspondante de régions dénudées hélicoïdales, isolées entre elles en direction circonférentielle par des enductions hélicoïdales de matériau électriquement non conducteur.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 montre le coeur et son réseau coronaire veineux dans lequel a été implantée une sonde selon l'invention.
La Figure 2 illustre un premier mode de réalisation de la partie active (celle liée au microcâble) de la sonde de l'invention.
La Figure 3 est une vue agrandie du détail repéré III sur la Figure 2.
La Figure 4 est une vue agrandie d'un second mode de réalisation de la partie active de la sonde de l'invention.
La Figure 5 est une caractéristique illustrant la manière dont l'on peut ajuster la surface active exposée par un choix approprié du diamètre d'enduction du matériau isolant de la partie active de la sonde illustrée Figure 4.
La Figure 6 est une variante du premier mode de réalisation de l'invention, dans lequel la sonde porte une pluralité de microcâbles distincts.

On va maintenant décrire des exemples de réalisation de la sonde selon l'invention.

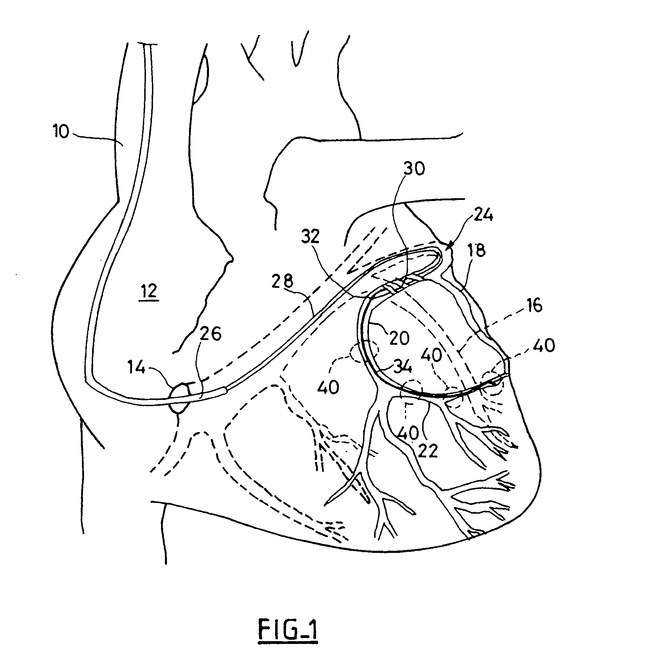

La Figure 1 illustre de façon générale le myocarde, dans lequel on a introduit une sonde selon l'invention, destinée à la stimulation du ventricule gauche.

Cette sonde est implantée par voie endocavitaire dans le réseau coronaire veineux via la veine cave supérieure 10, l'oreillette droite 12 et l'entrée 14 du sinus coronaire veineux.

Le réseau coronaire veineux se développe ensuite en plusieurs branches, comprenant les veines postéro-latérale 16, latérale 18, la grande veine cardiaque 20 et la veine antéro-latérale 22.

La référence 24 désigne de façon générale la sonde selon l'invention, qui comprend un corps de sonde avec une partie principale 26 (de diamètre 6 French par exemple) pénétrant jusque dans le sinus coronaire 14, prolongée par une partie de transition 28 de même conformation mais de diamètre plus réduit (par exemple 4,8 French) pour permettre une meilleure pénétration dans le réseau coronaire veineux.

Ce corps de sonde est formé d'une gaine creuse tubulaire en matériau déformable, par exemple en silicone ou polyuréthanne, définissant une lumière centrale d'une extrémité à l'autre du corps de sonde.

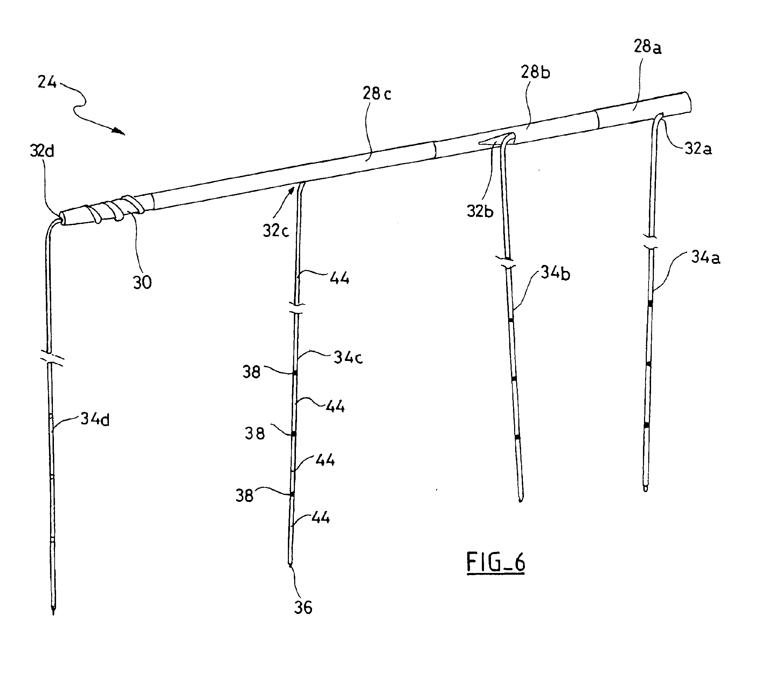

A l'extrémité distale, le corps de sonde est pourvu d'un moyen de retenue 30 permettant son maintien mécanique dans la veine. Ce moyen de retenue est par exemple constitué d'une vis en silicone telle que celle décrite dans le EP 1 374 945 A1 (ELA Medical), pourvue d'un filet hélicoïdal dont le diamètre extérieur maximal est de l'ordre de 7 French. Ce moyen de retenue 30 est également visible sur la Figure 6. Il s'agit d'un moyen du même type que celui utilisé par la sonde *Situs LV* précitée. Le filet est moulé sur un élément cylindrique terminant la partie de transition 28 du corps de sonde, le tout étant moulé monobloc en un matériau tel qu'un élastomère de silicone, matériau peu traumatique et assurant une bonne biocompatibilité. Par ailleurs, l'extrémité distale du corps de sonde, pourvue du moyen de retenue 30, est ouverte en 32, le débouché comportant des moyens d'étanchéité (non représentés, mais en eux-mêmes conventionnels), par exemple un bouchon percé en silicone, permettant d'éviter tout reflux de sang à l'intérieur du corps de sonde aussi bien en l'absence qu'en présence d'un élément introduit dans la lumière centrale du corps de sonde.

Ce corps de sonde est implanté par une technique conventionnelle de type OTW *(Over The Wire* ou "filoguidage") au moyen d'un mandrin très fin formant fil-guide, pourvu à son extrémité distale d'une terminaison très souple pour ne pas être traumatique et permettre son introduction directe dans les vaisseaux du réseau coronaire sans risque de perforation. Au préalable, le chirurgien dispose un cathéter principal lui permettant d'accéder au débouché du sinus coronaire, et un cathéter de sous-sélection permettant de choisir, sous amplificateur de brillance, le chemin du réseau veineux qui permettra d'atteindre la veine cible correspondant au site de stimulation choisi.

Le chirurgien introduit ensuite dans ce cathéter le fil-guide, qu'il pousse pour le faire progresser à nu dans le réseau veineux coronaire de façon à sélectionner telle ou telle veine collatérale. Une fois la veine sélectionnée, le chirurgien enfile sur le fil-guide le corps de sonde (le fil-guide traverse de façon étanche l'orifice 32 qui est normalement obturé par le bouchon percé). Il fait alors glisser et progresser le corps de sonde sur le fil-guide, qui guide axialement le corps de sonde jusqu'à l'emplacement choisi. Une fois le corps de sonde arrivé à l'emplacement définitif dans la veine choisie, le chirurgien imprime au corps de sonde un mouvement additionnel de rotation, ce qui assure, par effet de vissage du filet du moyen de retenue 30, la poursuite de la progression du corps de sonde sur quelques millimètres avec renforcement corrélatif de l'ancrage de celui-ci dans la veine.

De façon caractéristique, le corps de sonde que l'on vient de décrire (et dont la structure est en elle-même classique) est prolongé par un microcâble télescopique constituant la partie active 34 de la sonde (éventuellement en complément d'une électrode active de stimulation préexistante, disposée sur la sonde).

Ce microcâble présente un diamètre de l'ordre de 0,5 à 2 French et s'étend sur une longueur de 1 à 200 mm au-delà du débouché 32 de l'extrémité distale du corps de sonde.

Une fois le corps de sonde implanté de la manière que l'on a indiquée plus haut et après retrait du fil-guide, le microcâble est enfilé dans le corps de sonde à partir de l'extrémité proximale de celui-ci. Il est poussé sur toute la longueur du corps de sonde jusqu'à émerger du débouché 32 de l'extrémité proximale de ce dernier, puis est déployé au-delà de manière à le faire progresser, sous amplificateur de brillance, dans les veines collatérales jusqu'à atteindre la position souhaitée. Il est ainsi possible d'atteindre et de stimuler des zones du réseau coronaire veineux jusque là inaccessibles avec les sondes connues.

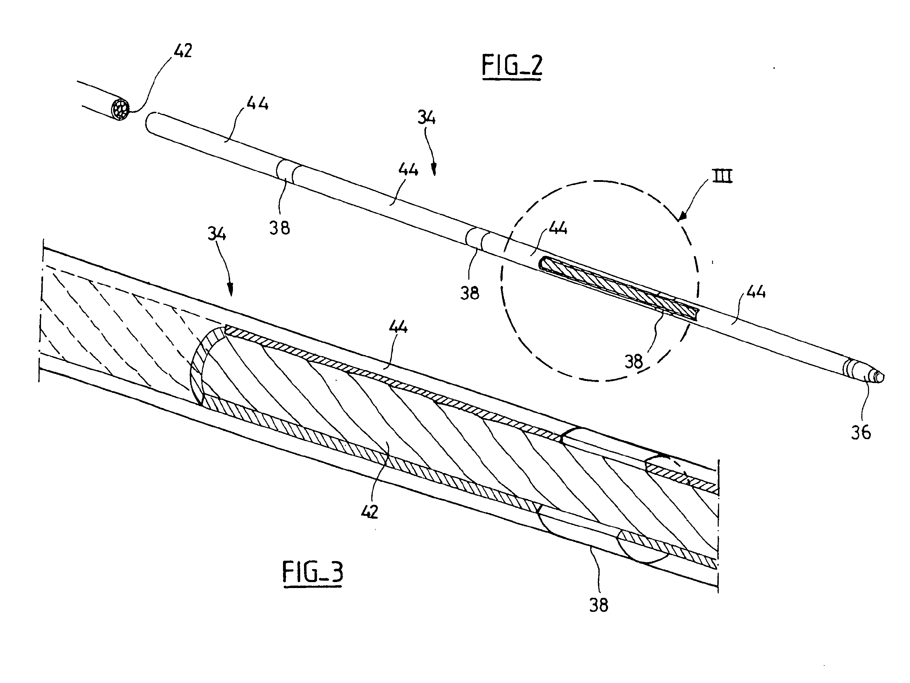

La partie active 34 du microcâble (c'est-à-dire sa partie émergeante) comporte une pluralité de parties dénudées formant une succession d'électrodes individuelles, constituant ensemble un réseau d'électrodes reliées en série permettant de multiplier les points de stimulation. Par exemple, sur la Figure 2, la partie active 34 comporte, outre l'électrode distale 36, une pluralité d'électrodes annulaires 38 disposées à intervalles réguliers sur la longueur de la partie active 34.

Ceci permet de multiplier les possibilités de points de contact 40 avec la paroi de la veine et d'assurer ainsi une diffusion multi-zone de l'énergie de stimulation en plusieurs points de l'épicarde et donc du ventricule gauche. Les Figures 2 et 3 illustrent un premier mode de réalisation de la partie active (celle liée à la partie émergeante du microcâble) de la sonde de l'invention.

L'âme de la partie active 34 est constituée par le microcâble 42, sur lequel sont enfilés successivement et alternativement des tubes isolants 44 et des électrodes conductrices courtes 38, le microcâble étant terminé par l'électrode distale 36.

Le microcâble 42 est avantageusement réalisé à base de nitinol (alliage NiTi), matériau dont l'avantage essentiel est son extrême endurance en fatigue.

Plus précisément, la structure du microcâble est une structure multifilaire dans laquelle chaque brin est constitué d'une âme en platine-iridium gainée par une épaisseur de nitinol (ou inversement), l'ensemble pouvant alors être revêtu :
- soit par une fine couche de parylène (par exemple de type C). Dans ce cas, des ouvertures plus ou moins complexes sont aménagées le long du microcâble, par exemple par ablation plasma, pour former les zones électriquement actives 36, 38. Pour améliorer les performances électriques, ces zones peuvent être en outre revêtues par exemple de nitrure de titane ;
- soit par un tube polyuréthanne.

Ces types de microcâbles sont disponibles par exemple auprès de la société Fort Wayne Metals Inc., Fort Wayne, USA, et sont utilisés dans le domaine médical notamment pour réaliser des conducteurs de défibrillation - toutefois selon un arrangement de matériaux différent : dans ces applications connues, la structure est une structure multifilaire dans laquelle chaque brin inclut une âme en argent (pour améliorer la conductivité) gainée par une épaisseur d'inox ; ces microstructures, isolées ou non, sont ensuite incorporées dans un corps de sonde multi-lumière de construction classique.

Les avantages de la structure de microcâble décrite plus haut résident dans le fait que les éléments les moins endurants mécaniquement (platine iridié ou argent) sont encapsulés directement dans la gaine en nitinol. Les conséquences d'une fracture éventuelle de ces brins sont donc minimisées.

Comme alternative, il est néanmoins possible de disposer un brin en platine-iridium au centre d'une structure multifilaire de type 1x7, le brin le plus fragile étant alors enlacé par les brins externes plus endurants.

Enfin, le platine-iridium peut être remplacé par tout matériau radio-opaque tel que le tantale, et le nitinol peut être remplacé par des matériaux moins performants en endurance mais néanmoins suffisants ou moins chers tels que l'acier inox MP35N, couramment utilisé dans la fabrication des conducteurs standard.

Les tubes isolants 44 séparant les électrodes 36, 38 sont avantageusement des tubes en PU (polyuréthanne), collés sur le microcâble avec une colle de type PU. La fluidité de cette colle, combinée aux anfractuosités formées par la torsade du microcâble, assure une liaison optimale entre tube PU et microcâble.

Les électrodes en platine 36 et 38 sont serties directement sur le microcâble. La faible épaisseur de l'électrode, associée à la ductilité du platine, favorise la qualité du contact électrique sans altérer le microcâble. D'autre part, la faible longueur des électrodes (0,5 à 10 mm) limite sensiblement leur impact sur le comportement mécanique global du système, qui sera principalement dicté par le microcâble.

La surface individuelle de chaque électrode est d'environ 0,5 mm², ce qui permet d'en disposer un nombre important (jusqu'à douze sur une longueur de 1 à 200 mm en direction longitudinale) sans dépasser une surface totale cumulée de 6 à 8 mm².

Du fait de la faible surface active cumulée, on bénéficie ainsi des avantages d'une sonde dite à "haute densité de courant", en termes à la fois d'efficacité physiologique de la stimulation et de moindre consommation d'énergie - ceci tout en maximisant les chances de contact physique, donc électrique, de la surface conductrice (électrodes 36 et 38) de la partie active 34 avec les tissus excitables, du fait de la multiplication des électrodes.

Par ailleurs, l'alternance de zone conductrices et isolantes combinée aux propriétés télescopiques du système permet de gérer au mieux les risques de stimulations phréniques. En effet, si pour une position donnée, le nerf phrénique est englobé dans le champs électrique, il est possible de faire coulisser le microcâble dans le corps de sonde jusqu'à positionner une zone isolée au droit du nerf et donc d'échapper à cette stimulation parasite.

La configuration que l'on vient de décrire permet de dissocier les problèmes liés au placement de la sonde dans le réseau coronaire veineux et ceux liés à la multiplication des points de stimulation.

En effet, la fixation et le maintien en place mécanique de la sonde sont assurés en amont par le corps de sonde proprement dit et par les moyens de retenue 30, tandis que la multiplication des points de stimulation est assurée par le réseau d'électrodes constitué sur le microcâble télescopique, ce qui permet de stimuler une large zone choisie indépendamment des contraintes habituelles d'accessibilité et de stabilité.

Par ailleurs, afin de favoriser le contact avec les tissus, de multiples types de préformes sont possibles pour l'extrémité distale du microcâble, notamment et de façon non limitative :
- enchainement de courbures à rayons variables dans un même plan ;
- enchainement de courbures à rayons variables dans une succession de plans distincts ;
- trajectoire tridimensionnelle fortement courbée, sans aucun plan de base, par exemple de type *pigtail* (queue de cochon),
   les dimensions externes de la préforme à l'état de repos étant comprises dans un cube de 1 à 35 mm de côté.

Cette configuration particulière permet notamment d'envisager une variante de pose du microcâble seul (sans la sonde), celui-ci étant alors maintenu en place par sa seule préforme distale. Dans ce cas, le placement dans la veine est réalisé par des moyens classiques de type cathéter/sous-cathéter ou cathéter d'accès cérébral.

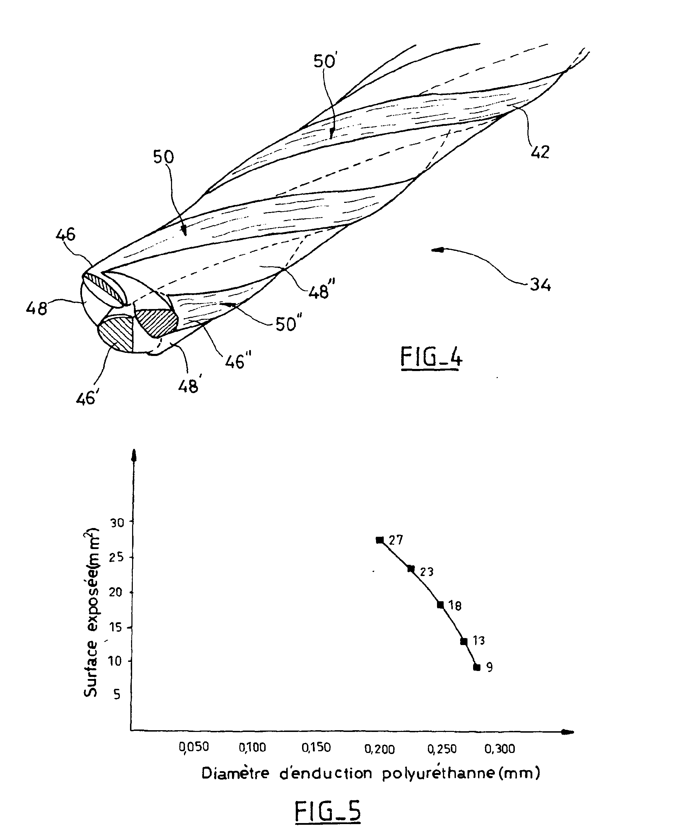

La Figure 4 illustre un second mode de réalisation de la partie active de la sonde de l'invention.

Dans ce mode de réalisation, la solution consiste à appliquer sur le toron formé par les fils 46, 46', 46" du microcâble une enduction de colle PU isolante 48, 48', 48", toutefois sur un diamètre inférieur au diamètre hors-tout du toron de manière à laisser apparaître, en réserve, des surfaces conductrices 50, 50', 50" (surfaces non enduites) de forme hélicoïdale.

La surface active de la partie active 34 est ainsi une surface en forme de triple hélice 50, 50', 50" en périphérie du microcâble, courant sur tout ou partie de la longueur de la région active 34

Cette solution permet d"'allonger" en direction longitudinale la surface de stimulation, sans augmenter pour autant la surface totale d'électrode. Plus précisément, le graphique de la Figure 5 illustre la manière d'ajuster la surface exposée totale (surface active) en fonction du diamètre de l'enduction de colle PU. Les chiffres de ce graphique sont donnés pour une longueur de 40 mm d'un microcâble à trois brins de diamètre hors-tout 0,3 mm.

Cette variante permet donc d'optimiser encore l'utilisation de la surface active d'électrode du microcâble, en favorisant son extension longitudinale. On notera que les régions hélicoïdales exposées 50, 50', 50" peuvent, si on le souhaite, n'apparaître que sur certaines régions du microcâble, en faisant par exemple alterner des régions actives où les surfaces exposées hélicoïdales sont apparentes à des régions complètement isolées, par exemple au moyen de tubes PU tels que les tubes 44 décrits à propos du premier mode de réalisation décrit plus haut.

D'autre part, les zones conductrices dénudées du microcâble peuvent recevoir un revêtement de type poreux, par exemple de type NiTi, ou être revêtus d'une couche additionnelle formée par un film de carbone déposé par pulvérisation cathodique, pour améliorer les propriétés de biocompatibilité entre le microcâble, son isolant et son environnement, de manière à éviter toute dégradation des parties en contact avec un flux sanguin. Les US 5 370 684 A et US 5 387 247 A, au nom de Sorin Biomedica SpA, décrivent la manière de déposer par pulvérisation cathodique un film mince, submicronique, de carbone sur des prothèses implantables telles que cathéters, valves cardiaques, etc. en polyuréthanne ou silicone. On se réfèrera à ces deux documents pour plus de détails sur la technologie permettant de réaliser ce dépôt. La Figure 6 illustre une variante du premier mode de réalisation de l'invention, dans lequel la sonde porte une pluralité de microcâbles distincts dont chacun est semblable à celui qui a été décrit plus haut et illustré sur les Figures 2 et 3.

Ici, un même corps de sonde est doté de sonde de plusieurs lumières internes parallèles desquelles émergent latéralement une pluralité de microcâbles respectifs.

Ainsi, sur la Figure 6, le corps de sonde comporte une pluralité de sections 28a, 28b, 28c pourvues chacune d'un orifice 32a, 32b, 32c communiquant avec une lumière respective. Il comporte également à son extrémité distale les moyens de retenue 30 ainsi que l'orifice 32d.

De chacun des orifices 32a, 32b, 32c, 32d émerge un microcâble respectif 34a, 34b, 34c, 34d réalisé selon l'un ou l'autre des modes de réalisation exposés plus haut, par exemple selon le premier mode de réalisation dans l'exemple illustré Figure 6. Chacun de ces microcâbles porte un réseau propre d'électrodes 36, 38 réunies électriquement ensemble et séparées par des parties isolantes 44, sur toute la longueur de la partie émergeante.

Cette solution permet de couvrir une large surface du ventricule gauche, avec une multitude d'électrodes connectées en parallèle, chaque branche correspondant à chaque partie active 34a, 34b, 34c, 34d restant toutefois électriquement indépendante grâce à une liaison électrique propre jusqu'à l'extrémité proximale de la sonde. Cette liaison permet le raccordement à une borne correspondante d'un connecteur multiple, par exemple de type IS-4. Il peut être également prévu à l'intérieur de la sonde un système de multiplexage intégré permettant de gérer séparément les divers réseaux d'électrodes.

De façon générale, quel que soit le mode de réalisation envisagé, la technique que l'on vient d'exposer présente de nombreux avantages, parmi lesquels on peut citer en particulier :
- méthode de pose simplifiée, ne nécessitant qu'un matériel conventionnel ;
- stabilité du contact électrique avec les tissus indépendamment de la taille de la veine ;
- possibilité d'élargir la partie exploitable de la veine, notamment vers la zone distale du réseau veineux, avec une excellente adaptation aux réseaux veineux fins, tout en gardant un point de fixation dans la veine cible (au niveau des moyens de maintien 30) ;
- bonne diffusion du flux électrique dans les régions profondes de l'épicarde ;
- fiabilité élevée, grâce aux performances mécaniques de la structure en nitinol du microcâble ;
- simplicité mécanique d'ensemble, donc faible coût de fabrication et fiabilité élevée ;
- radio-opacité, grâce à l'âme en platine de chaque brin formant le microcâble ainsi que par les bagues en platine (dans le cas du premier mode de réalisation) ;
- extraction aisée, grâce (i) au profil isodiamètre du microcâble, (i) à son faible diamètre et (iii) à sa forte résistance à la traction (structure monobloc robuste du microcâble jusqu'à son extrémité) ; pour le corps de sonde, il suffira de dévisser l'extrémité distale du corps de sonde au niveau des moyens de maintien 30 avant de retirer le corps de sonde.

## Revendications

1. Une sonde de stimulation, destinée à être implantée dans une veine du réseau coronarien (14-22) pour la stimulation d'une cavité gauche du coeur,
cette sonde comprenant un câble télescopique (42) en matériau conducteur, comportant à son extrémité distale une partie libre active (34) comprenant une pluralité de régions dénudées (36, 38 ; 50, 50', 50") distinctes,
ces régions dénudées étant destinées à venir en contact (40) avec la paroi d'une veine cible (22) du réseau coronaire, de manière à constituer ainsi un réseau d'électrodes de stimulation,
le câble comprenant en outre, côté proximal, des moyens de couplage à un générateur de dispositif médical implantable actif tel qu'un stimulateur cardiaque ou resynchroniseur,
sonde **caractérisée en ce que** :
- le câble télescopique est un microcâble, dont le diamètre est compris entre 0,167 et 0,667 mm (0,5 et 2 French);
- ce microcâble est formé d'une pluralité de brins toronnés ensemble, dont au moins certains sont des brins incorporant une âme en matériau radio-opaque de type platine-iridium ou tantale enveloppée dans une gaine en matériau mécaniquement endurant de type NiTi ou inox, ou inversement ; et
- les régions dénudées distinctes (36, 38 ; 50, 50', 50") sont des régions dénudées du microcâble, et forment un réseau d'électrodes de stimulation reliées électriquement ensemble.

2. La sonde de la revendication 1, dans laquelle l'extrémité distale du microcâble présente une préforme bi- ou tridimensionnelle.

3. La sonde de la revendication 2, dans laquelle les dimensions externes de la préforme à l'état de repos sont comprises dans un cube de 1 à 90 mm de côté.

4. La sonde de la revendication 2, dans laquelle ladite préforme d'extrémité distale du microcâble comprend un enchaînement de courbures à rayons variables dans un même plan.

5. La sonde de la revendication 2, dans laquelle ladite préforme d'extrémité distale du microcâble comprend un enchaînement de courbures à rayons variables dans une succession de plans distincts.

6. La sonde de la revendication 2, dans laquelle ladite préforme d'extrémité distale du microcâble comprend une trajectoire tridimensionnelle fortement courbée sans aucun plan de base.

7. La sonde de la revendication 6, dans laquelle ladite trajectoire tridimensionnelle fortement courbée est de type *pigtail,* queue de cochon..

8. La sonde de la revendication 6, dans laquelle les dimensions externes de ladite préforme d'extrémité distale du microcâble à l'état de repos sont comprises dans un cube de 1 à 35 mm de côté.

9. La sonde de la revendication 1, comprenant en outre un corps de sonde comportant une gaine creuse (26, 28) en matériau déformable, avec une lumière centrale ouverte à ses deux extrémités et dans laquelle le microcâble est disposé à coulissement en s'étendant sur toute la longueur du corps de sonde et au-delà de l'extrémité distale (32) de celui-ci, la partie du microcâble émergeant au-delà de l'extrémité distale du corps de sonde formant ladite une partie libre active (34).

10. La sonde de la revendication 9, dans laquelle l'extrémité distale du corps de sonde comporte des moyens de retenue (30) comprenant au moins un relief formé sur le corps de sonde et présentant localement un diamètre accru par rapport au diamètre propre du corps de sonde.

11. La sonde de la revendication 10, dans laquelle le relief est un relief hélicoïdal avec un filet s'enroulant autour du corps de sonde.

12. La sonde de la revendication 10, dans laquelle le diamètre accru du relief de retenue est inférieur ou égal à 2,333 mm (7 French).

13. La sonde de la revendication 9, dans laquelle le corps de sonde comporte une partie principale (26) prolongée côté distal par une partie de transition (28) de diamètre inférieur à celui de la partie principale.

14. La sonde de la revendication 9, dans laquelle le diamètre de la partie principale est inférieur ou égal à 2 mm (6 French), et celui de la partie de transition est inférieur ou égal à 1,667 mm (5 French).

15. La sonde de la revendication 9, comprenant :
- un corps de sonde (28a, 28b, 28c) commun ; et
- une pluralité de microcâbles télescopiques distincts, chacun étant logé dans le corps de sonde et apte à coulisser dans celui-ci, les parties libres actives respectives (34a, 34b, 34c, 34d) des différents microcâbles émergeant du corps de sonde en des emplacement distincts (32a, 32b, 32c, 32d), espacés longitudinalement sur le corps de sonde.

16. La sonde de la revendication 1, dans laquelle la surface totale exposée de la (des) région(s) dénudée(s) de la partie libre active du microcâble est d'au moins 1 mm², de préférence comprise entre 4 et 6 mm².

17. La sonde de la revendication 1, dans laquelle la longueur de la partie libre active du microcâble est réglable entre 1 et 200 mm.

18. La sonde de la revendication 1, dans laquelle la partie libre active du microcâble comporte une pluralité de régions dénudées distinctes (36, 38) s'étendant en succession le long de cette partie libre active.

19. La sonde de la revendication 18, dans laquelle les régions dénudées distinctes (36, 38) sont séparées les unes des autres par des parties de tube (44) en matériau électriquement non conducteur, enveloppant et gainant le microcâble entre deux régions dénudées consécutives.

20. La sonde de la revendication 18, dans laquelle les régions dénudées portent des bagues tubulaires en matériau électriquement conducteur, serties sur le microcâble.

21. La sonde de la revendication 20, dans laquelle le matériau électriquement conducteur des bagues tubulaires serties sur le microcâble est un matériau radio-opaque.

22. La sonde de la revendication 18, dans laquelle le microcâble comprend une structure multifilaire revêtue par un matériau isolant, notamment par du parylène, dans lequel les régions dénudées sont formées en ménageant des ouvertures par ablation le long du microcâble, et dépôt éventuel de nitrure de titane sur les régions dénudées ainsi formées.

23. La sonde de la revendication 18, dans laquelle la longueur en direction longitudinale de chaque région dénudée est comprise entre 0,5 et 10 mm.

24. La sonde de la revendication 1, dans laquelle la partie libre active du microcâble comporte au moins une région dénudée hélicoïdale (50, 50', 50") s'étendant le long de cette partie libre active.

25. La sonde de la revendication 24, dans laquelle le microcâble comporte, sur au moins une portion de la partie libre active, un toron formé d'une pluralité de brins torsadés (46, 46', 46") présentant en surface une pluralité correspondante de régions dénudées hélicoïdales (50, 50', 50"), isolées entre elles en direction circonférentielle par des enductions hélicoïdales (48, 48', 48") de matériau électriquement non conducteur.

## Claims

1. Stimulation probe, intended to be implanted in a vein of the coronary network (14-22) for the stimulation of a left cavity of the heart,
this probe comprising a telescopic cable (42) made of conductive material, comprising at its distal end an active free part (34) comprising an plurality of distinct stripped regions (36, 38; 50, 50', 50"),
these stripped regions being intended to come into contact (40) with the wall of a target vein (22) of the coronary network, so as to thus form a network of stimulation electrodes,
the cable also comprising, at the proximal end, means for coupling to a generator of an active implantable medical device such as a cardiac stimulator or resynchronizer,
the probe being **characterized in that**:
- the telescopic cable is a microcable, the diameter of which is between 0.167 and 0.667 mm (0.5 and 2 French);
- this microcable is formed from a plurality of strands twisted together, at least some of which are strands incorporating a core made of radio-opaque material of platinum-iridium or tantalum type jacketed in a sheath made of mechanically resistant material of NiTi or stainless type, or vice versa; and
- the distinct stripped regions (36, 38; 50, 50', 50") are stripped regions of the microcable, and form a network of stimulation electrodes electrically linked together.

2. Probe of Claim 1, in which the distal end of the microcable has a two- or three-dimensional preform.

3. Probe of Claim 2, in which the external dimensions of the preform in the rest state are contained within a cube of 1 to 90 mm side.

4. Probe of Claim 2, in which said distal end preform of the microcable comprises a sequence of curves with variable radii in one and the same plane.

5. Probe of Claim 2, in which said distal end preform of the microcable comprises a sequence of curves with variable radii in a succession of distinct planes.

6. Probe of Claim 2, in which said distal end preform of the microcable comprises a three-dimensional trajectory that is strongly curved with no base plane.

7. Probe of Claim 6, in which said strongly curved three-dimensional trajectory is of pigtail type.

8. Probe of Claim 6, in which the external dimensions of said distal end preform of the microcable in the rest state are contained within a cube of 1 to 35 mm side.

9. Probe of Claim 1, also comprising a probe body comprising a hollow sheath (26, 28) made of a deformable material, with a central opening open at its two ends and in which the microcable is arranged to slide by extending over the entire length of the probe body and beyond the distal end (32) thereof, the part of the microcable emerging beyond the distal end of the probe body forming said one active free part (34).

10. Probe of Claim 9, in which the distal end of the probe body comprises retaining means (30) comprising at least one relief formed on the probe body and having, locally, an increased diameter compared to the specific diameter of the probe body.

11. Probe of Claim 10, in which the relief is a helical relief with a thread winding around the probe body.

12. Probe of Claim 10, in which the increased diameter of the retaining relief is less than or equal to 2.333 mm (7 French).

13. Probe of Claim 9, in which the probe body comprises a main part (26) prolonged at the distal end by a transition part (28) of diameter less than that of the main part.

14. Probe of Claim 9, in which the diameter of the main part is less than or equal to 2 mm (6 French), and that of the transition part is less than or equal to 1.667 mm (5 French).

15. Probe of Claim 9, comprising:
- a common probe body (28a, 28b, 28c); and
- a plurality of distinct telescopic microcables, each being housed in the probe body and capable of sliding therein, the respective active free parts (34a, 34b, 34c, 34d) of the different microcables emerging from the probe body at distinct places (32a, 32b, 32c, 32d), spaced apart longitudinally on the probe body.

16. Probe of Claim 1, in which the total surface area exposed to the stripped region(s) of the active free part of the microcable is at least 1 mm², preferably between 4 and 6 mm².

17. Probe of Claim 1, in which the length of the active free part of the microcable can be adjusted between 1 and 200 mm.

18. Probe of Claim 1, in which the active free part of the microcable comprises a plurality of distinct stripped regions (36, 38) extending in succession along this active free part.

19. Probe of Claim 18, in which the distinct stripped regions (36, 38) are separated from one another by tube parts (44) made of electrically nonconductive material, jacketing and sheathing the microcable between two consecutive stripped regions.

20. Probe of Claim 18, in which the stripped regions bear tubular rings made of electrically conductive material, crimped onto the microcable.

21. Probe of Claim 20, in which the electrically conductive material of the tubular rings crimped onto the microcable is a radio-opaque material.

22. Probe of Claim 18, in which the microcable comprises a multi-wire structure coated by an insulating material, notably by parylene, in which the stripped regions are formed by creating openings by ablation along the microcable, and possible deposition of titanium nitride on the duly formed stripped regions.

23. Probe of Claim 18, in which the length in the longitudinal direction of each stripped region is between 0.5 and 10 mm.

24. Probe of Claim 1, in which the active free part of the microcable comprises at least one helical stripped region (50, 50', 50") extending along this active free part.

25. Probe of Claim 24, in which the microcable comprises, on at least one portion of the active free part, a loom formed by a plurality of twisted strands (46, 46', 46") having on the surface a corresponding plurality of helical stripped regions (50, 50', 50"), insulated from one another in the circumferential direction by helical coatings (48, 48', 48") of electrically nonconductive material.

## Patentansprüche

1. Stimulationssonde, die dazu bestimmt ist, in eine Vene des koronaren Venennetzes (14-22) zur Stimulation einer linken Herzkammer implantiert zu werden,
wobei diese Sonde ein Teleskopkabel (42) aus leitendem Material enthält, das an seinem distalen Ende einen aktiven freien Teil (34) aufweist, der eine Vielzahl von verschiedenen abisolierten Zonen (36, 38; 50, 50', 50") enthält,
wobei diese abisolierten Zonen dazu bestimmt sind, mit der Wand einer Zielvene (22) des Koronarvenennetzes in Kontakt zu kommen (40), um so ein Netz von Stimulationselektroden zu bilden, wobei das Kabel außerdem auf der proximalen Seite Einrichtungen zur Kopplung mit einem Generator einer aktiven implantierbaren medizinischen Vorrichtung, wie einem Herzschrittmacher oder Resynchronisierer, enthält,
wobei die Sonde **dadurch gekennzeichnet ist, dass**:
- das Teleskopkabel ein Mikrokabel ist, dessen Durchmesser zwischen 0,167 und 0,667 mm (0,5 und 2 French) liegt;
- dieses Mikrokabel von einer Vielzahl von miteinander verdrillten Adern geformt wird, von denen mindestens bestimmte Adern sind, die einen Kern aus röntgendichtem Material von der Art Platin-Iridium oder Tantal enthalten, eingehüllt in eine Hülle aus mechanisch ausdauerndem Material der Art NiTi oder rostfreiem Stahl, oder umgekehrt; und
- die verschiedenen abisolierten Zonen (36, 38; 50, 50', 50") abisolierte Zonen des Mikrokabels sind und ein Netz von Stimulationselektroden formen, die elektrisch miteinander verbunden sind.

2. Sonde nach Anspruch 1, wobei das distale Ende des Mikrokabels eine zwei- oder dreidimensionale Preform aufweist.

3. Sonde nach Anspruch 2, wobei die Außenabmessungen der Preform im Ruhezustand in einem Kubus von 1 bis 90 mm Seitenlänge enthalten sind.

4. Sonde nach Anspruch 2, wobei die Preform des distalen Endes des Mikrokabels eine Verkettung von Krümmungen mit variablen Radien in der gleichen Ebene enthält.

5. Sonde nach Anspruch 2, wobei die Preform des distalen Endes des Mikrokabels eine Verkettung von Krümmungen mit variablen Radien in einer Folge von verschiedenen Ebenen enthält.

6. Sonde nach Anspruch 2, wobei die Preform des distalen Endes des Mikrokabels einen dreidimensionalen stark gekrümmten Verlauf ohne Basisebene enthält.

7. Sonde nach Anspruch 6, wobei der stark gekrümmte Verlauf von der Art Pigtail, Schweineschwanz, ist.

8. Sonde nach Anspruch 6, wobei die Außenabmessungen der Preform des distalen Endes des Mikrokabels im Ruhezustand in einem Kubus mit einer Seitenlänge von 1 bis 35 mm enthalten sind.

9. Sonde nach Anspruch 1, die außerdem einen Sondenkörper enthält, der eine Hohlumhüllung (26, 28) aus verformbarem Material mit einer zentralen Öffnung enthält, die an ihren beiden Enden offen ist, und in der das Mikrokabel gleitend angeordnet ist, indem es sich über die ganze Länge des Sondenkörpers und über dessen distales Ende (32) hinaus erstreckt, wobei der Teil des Mikrokabels, der aus dem distalen Ende des Sondenkörpers austritt, den aktiven freien Teil (34) formt.

10. Sonde nach Anspruch 9, wobei das distale Ende des Sondenkörpers Halteeinrichtungen (30) aufweist, die mindestens ein Relief enthalten, das auf dem Sondenkörper geformt ist und lokal einen bezüglich des Eigendurchmessers des Sondenkörpers erhöhten Durchmesser aufweist.

11. Sonde nach Anspruch 10, wobei das Relief ein spiralförmiges Relief mit einem Gewindegang ist, der sich um den Sondenkörper windet.

12. Sonde nach Anspruch 10, wobei der erhöhte Durchmesser des Haltereliefs geringer als oder gleich 2,333 mm (7 French) ist.

13. Sonde nach Anspruch 9, wobei der Sondenkörper einen Hauptteil (26) aufweist, der auf der distalen Seite durch einen Übergangsteil (28) verlängert wird, dessen Durchmesser geringer ist als derjenige des Hauptteils.

14. Sonde nach Anspruch 9, wobei der Durchmesser des Hauptteils geringer als oder gleich 2 mm (6 French) und derjenige des Übergangsteils geringer als oder gleich 1,667 mm (5 French) ist.

15. Sonde nach Anspruch 9, die enthält:
- einen gemeinsamen Sondenkörper (28a, 28b, 28c); und
- eine Vielzahl von verschiedenen Teleskop-Mikrokabeln, die je im Sondenkörper untergebracht sind und in diesem gleiten können, wobei die jeweiligen aktiven freien Teile (34a, 34b, 34c, 34d) der unterschiedlichen Mikrokabel aus dem Sondenkörper an verschiedenen Stellen (32a, 32b, 32c, 32d) austreten, die in Längsrichtung auf dem Sondenkörper beabstandet sind.

16. Sonde nach Anspruch 1, wobei die gesamte freiliegende Fläche der abisiolierten Zone(n) des aktiven freien Teils des Mikrokabels mindestens 1 mm² beträgt, vorzugsweise zwischen 4 und 6 mm² liegt.

17. Sonde nach Anspruch 1, wobei die Länge des aktiven freien Teils des Mikrokabels zwischen 1 und 200 mm einstellbar ist.

18. Sonde nach Anspruch 1, wobei der aktive freie Teil des Mikrokabels eine Vielzahl von verschiedenen abisolierten Zonen (36, 38) enthält, die sich aufeinanderfolgend entlang dieses aktiven freien Teils erstrecken.

19. Sonde nach Anspruch 18, wobei die verschiedenen abisolierten Zonen (36, 38) voneinander durch Rohrteile (44) aus elektrisch nicht leitendem Material getrennt sind, die das Mikrokabel zwischen zwei aufeinanderfolgenden abisolierten Zonen umhüllen und ummanteln.

20. Sonde nach Anspruch 18, wobei die abisolierten Zonen rohrförmige Ringe aus elektrisch leitendem Material tragen, die auf das Mikrokabel gequetscht sind.

21. Sonde nach Anspruch 20, wobei das elektrisch leitende Material der auf das Mikrokabel gequetschten rohrförmigen Ringe ein röntgendichtes Material ist.

22. Sonde nach Anspruch 18, wobei das Mikrokabel eine mehrdrahtige Struktur, die mit einem Isoliermaterial, insbesondere Parylen, bedeckt ist, in dem die abisolierten Zonen durch Aussparen von Öffnungen durch Abtragen entlang des Mikrokabels geformt werden, und eine mögliche Ablagerung von Titannitrid auf den so geformten abisolierten Zonen enthält.

23. Sonde nach Anspruch 18, wobei die Länge in Längsrichtung jeder abisolierten Zone zwischen 0,5 und 10 mm liegt.

24. Sonde nach Anspruch 1, wobei der aktive freie Teil des Mikrokabels mindestens eine spiralförmige abisolierte Zone (50, 50', 50") aufweist, die sich entlang dieses aktiven freien Teils erstreckt.

25. Sonde nach Anspruch 24, wobei das Mikrokabel über mindestens einen Abschnitt des aktiven freien Teils eine von einer Vielzahl von verdrillten Adern (46, 46', 46") geformte Litze aufweist, die an der Oberfläche eine entsprechende Vielzahl von spiralförmigen abisolierten Zonen (50, 50', 50") aufweist, die untereinander in Umfangsrichtung durch spiralförmige Beschichtungen (48, 48', 48") eines elektrisch nicht leitenden Materials isoliert sind.
